# EUROPEAN PATENT APPLICATION

(11) **EP 3 295 861 A1**
(43) Date of publication of application: **21.03.2018**
(21) Application number: 16796044.2
(22) Date of filing: 11.03.2016
(51) Int. Cl.: A61B 1/06, G02B 23/26, H01L 33/00, H01S 5/0683

(54) **LIGHT SOURCE DEVICE, LIGHT SOURCE DRIVING METHOD, AND OBSERVATION DEVICE**

(30) Priority: 15.05.2015 JP 2015099880
(71) Applicant: Sony Corporation, Tokyo 108-0075 (JP)
(72) Inventor: MURAMATSU, Hirotaka, Tokyo 108-0075 (JP)
(74) Representative: D Young & Co LLP
(86) International application number: PCT/JP2016/001390
(87) International publication number: WO 2016/185647

(57) **Abstract**

A light source apparatus according to an embodiment of the present technology includes a white light source, a plurality of laser light sources, a detection unit, and a light source control unit. The white light source emits white light. The plurality of laser light sources emit a plurality of laser lights capable of adjusting a color temperature of the white light. The detection unit is capable of detecting a failure of each of the white light source and the plurality of laser light sources. The light source control unit controls an intensity ratio of the plurality of laser lights that are emitted from the plurality of laser light sources in a case where the failure of the white light source is detected.

## Description

### Technical Field

The present technology relates to a light source apparatus, a light source driving method, and an observation apparatus.

### Background Art

In recent years, as a light source of an observation apparatus, which is used to observe an operating field of a patient, such as an endoscope apparatus and a microscope apparatus, laser is increasingly used in place of a lamp light source that has been widely used. Advantages of using the laser as the light source are as follows: For example, a low power consumption is expected because electricity to light conversion efficiency of the light source is high, and enhanced observation of specific tissues is easily performed by combining light absorption properties of tissues such as blood vessels because wavelength bands are narrow.

For example, Patent Literature 1 discloses an endoscope system including a bluish violet laser light source (405DL) used as a special light source, and a blue laser light source (445LD) used as a light source of white light illumination light. As shown in Fig. 2 of Patent Literature 1, main lights (33a and 35a) and spare lights (33b and 35b) are provided for each of the bluish violet laser light source and the blue laser light source (paragraph [0039], etc. of the specification of Patent Literature 1).

In the paragraphs [0120]-[0136] of the specification and Fig. 10 to Fig. 12, etc. of Patent Literature 1, there is a description about a control method in a case where any of laser light sources has a failure and the light is turned off. In particular, in a case where both of the main light and the spare light (35a and 35b) of the blue laser light source as a light source of a white light illumination light have failures, bright illumination light is not provided. Therefore, an alarm display such as "immediately draw off the scope" is issued and it informs that there is no other choice.

### Citation List

### Patent Literature

Patent Literature 1: 2014-240021

### Disclosure of Invention

### Technical Problem

It is desirable to provide a technology that an effect on an operator's operation can be suppressed even in a case where a mounted light source has a failure, as described above.

The present technology is made in view of the above-mentioned circumstances, and it is an object of the present technology to provide a light source apparatus, a light source driving method, and an observation apparatus that can sufficiently suppress an effect of a light source failure.

### Solution to Problem

In order to achieve the object, a light source apparatus according to an embodiment of the present technology includes a white light source, a plurality of laser light sources, a detection unit, and a light source control unit.

The white light source emits white light.

The plurality of laser light sources emit a plurality of laser lights capable of adjusting a color temperature of the white light.

The detection unit is capable of detecting a failure of each of the white light source and the plurality of laser light sources.

The light source control unit controls an intensity ratio of the plurality of laser lights that are emitted from the plurality of laser light sources in a case where the failure of the white light source is detected.

In this light source apparatus, the plurality of laser light sources emit a plurality of laser lights for adjusting a color temperature of the white light. In a case where the failure of the white light source is detected, an intensity ratio of the plurality of laser lights is controlled. Thus, instead of the white light from the white light source, the plurality of laser lights of which the intensity ratio is controlled as appropriate are emitted from the white light source. As a result, it is possible to sufficiently suppress an effect of a white light source failure.

The light source control unit may emit the white light from the white light source in a case where the failure of any of the plurality of laser light sources is detected.

Thus, it is possible to sufficiently suppress an effect of a laser light source failure.

The light source apparatus may further include a filter unit and a multiplex unit.

The filter unit attenuates each of wavelength band components of the plurality of laser lights.

The multiplex unit multiplexes the white light and the plurality of laser lights, the white light being the white light where each of wavelength band components of the plurality of laser lights is attenuated by the filter unit.

Thus, the color temperature can be controlled with high accuracy.

The plurality of laser light sources may include a first laser light source and a second laser light source different from the first laser light source. In this case, the light source control unit drives only the white light source in a case where the failure of the first laser light source is detected, and drives the first laser light source and the white light source in a case where the failure of the second laser light source is detected.

Thus, it is possible to control by dealing with the failures of the respective first and second laser light sources and to improve the operability of the light source apparatus.

The light source apparatus may further includes a filter driving unit capable of removing the filter unit from a light path of the white light emitted from the white light source in a case where the failure of the first laser light source is detected.

Thus, in a case where the first laser light source has a failure, the white light including the components not attenuated can be emitted.

The white light source may be a white LED (Light Emitting Diode).

In this case, the first laser light source of the plurality of laser light sources may include a blue laser light source, and the second laser light source of the plurality of laser light sources may include a red laser light source and a green laser light source.

Thus, even in a case where any light source has a failure, it is possible to emit the white light and to sufficiently suppress the effect on the operator's operation.

A light source driving method according to an embodiment of the present technology includes detecting a failure of each of a white light source that emits white light and a plurality of laser light sources that emit a plurality of laser lights capable of adjusting a color temperature of the white light, and controlling an intensity ratio of the plurality of laser lights that are emitted from the plurality of laser lights in a case where the failure of the white light source is detected.

In a case where the failure of the white light source is detected, the intensity ratio of the plurality of laser lights that are emitted from the plurality of laser lights is controlled.

The light source driving method may further include multiplexing attenuated white light and the plurality of laser lights, the attenuated white light being the white light where each of wavelength band components of the plurality of laser lights is attenuated, in a case where no failure of the white light source and the plurality of laser light sources is detected.

The light source driving method may further includes driving only the white light source in a case where the failure of a first laser light source among the plurality of laser light sources is detected, and multiplexing and emitting the attenuated white light and the laser light emitted from the first laser light source in a case where the failure of second laser light source is detected, the second laser light source being different from the first laser light source among the plurality of laser light sources.

The light source driving method further includes removing a filter unit that attenuates each of wavelength band components of the plurality of laser lights from a light path of the white light emitted from the white light source in a case where the failure of the first laser light source is detected.

An observation apparatus according to an embodiment of the present technology includes the light source apparatus and an irradiation unit.

The irradiation unit irradiates a region that is a target to be observed with light emitted from the light source apparatus. Advantageous Effects of Invention

As described above, according to the present technology, the effect of the light source failure can be sufficiently suppressed. It should be noted that the effects described here are not necessarily limitative and may be any of effects described in the present disclosure.

### Brief Description of Drawings

[Fig. 1] Fig. 1 is a block diagram showing a configuration example of an endoscope apparatus according to a first embodiment of the present technology.
[Fig. 2] Fig. 2 is a schematic diagram showing a specific configuration example of a light source unit shown in Fig. 1.
[Fig. 3] Fig. 3 is a graph showing an example of a spectrum of white light emitted from a first light emitting unit.
[Fig. 4] Fig. 4 is a graph showing an example of a spectrum of laser multiplexed light emitted from a second light emitting unit.
[Fig. 5] Fig. 5 is a graph showing an example of a reflection property of a dichroic mirror dichroic mirror (filter unit).
[Fig. 6] Fig. 6 is a graph showing an example of a spectrum of white light reflected by a dichroic mirror dichroic mirror (filter unit).
[Fig. 7] Fig. 7 is a graph showing an example of a spectrum of multiplexed white light.
[Fig. 8] Fig. 8 is a flow chart showing an example of control in a case of failure of respective light sources.
[Fig. 9] Fig. 9 shows tables each indicating an example of intensity ratios of lights output from the remaining non-failed light sources.
[Fig. 10] Fig. 10 is a graph showing an example of a spectrum of multiplexed light of attenuated white light and blue laser light.
[Fig. 11] Fig. 11 is a schematic diagram showing a configuration example of a light source apparatus according to a second embodiments. Mode(s) for Carrying Out the Invention

Hereinafter, embodiments of the present technology will be described with reference to the drawings.

### <First embodiment>

### [Endoscope apparatus]

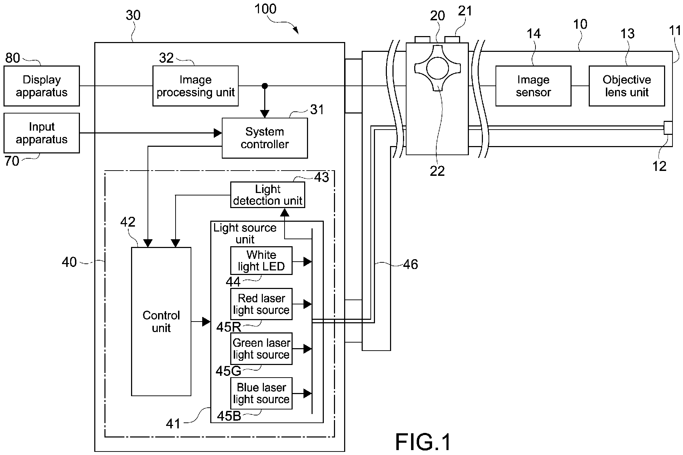

Fig. 1 is a block diagram showing a configuration example of an endoscope apparatus that is an observation apparatus according to a first embodiment of the present technology. The endoscope apparatus 100 includes an insert unit 10, an operation unit 20, and a main unit 30.

The insert unit 10 is a section that is inserted into a body cavity and has flexibility. An irradiation port 12 for irradiating a region that is a target to be observed (region to be observed) with illumination light is provided for a front portion 11 of the insert unit 10. Also, an objective lens unit 13 and an image sensor 14 are arranged at the front portion 11 in order to acquire image information of the region to be observed. As the image sensor 14, a CMOS (Complementary Metal-Oxide Semiconductor) sensor, a CCD (Charge Coupled Device) sensor, or the like is used, for example.

The operation unit 20 is connected to a base end of the insert unit 10. A variety of switches 21, an angle knob 22, and the like are arranged at the operation unit 20. For example, when a switch 21 is operated, air, water, or the like is fed to the region to be observed via an inlet (not shown). In addition, when the angle knob 22 is operated, a bending mechanism arranged at the front portion 11 moves, and the front portion 11 is bent in an arbitrary direction or an arbitrary angle. A forceps opening into which a treatment tool such as forceps and an electrode is inserted may be provided for the operation unit 20.

The main unit 30 includes a system controller 31, an image processing unit 32, and a light source apparatus 40 according to the present technology. The system controller 31 supervises and controls an overall system of the endoscope apparatus 100. For example, the system controller 31 instructs the light source apparatus 40 to emit a predetermined illumination light, and causes the image sensor 14 to acquire the image information of the region to be observed being synchronous therewith.

Also, as shown in Fig. 1, an input apparatus 70 is connected to the system controller 31 via an input/output interface (not shown). The input apparatus 70 is a device such as a keyboard, a mouse, and a touch panel that an operator operates. The system controller 31 executes processing corresponding to the operation input via the input apparatus 70.

The system controller 31 includes a CPU, a RAM, a ROM, and the like, for example, and the CPU loads a control program prerecorded in the ROM to the RAM and executes the program to thereby control the overall system. The configuration of the system controller 31 is not limited, and arbitrary hardware and software may be used. For example, a device such as an FPGA (Field Programmable Gate Array) and ASIC (Application Specific Integrated Circuit) may be used.

The image processing unit 32 executes a variety of image processing such as color interpolation and a gamma correction on the image information output from the image sensor 14. The image information processed by the image processing unit 32 is output to a display apparatus 80 connected to the main unit 30. The display apparatus 80 is a display device using liquid crystal, EL (ElectroLuminescence), or the like, for example.

### [Light source apparatus]

The light source apparatus 40 includes a light source unit 41, a control unit 42, and a light detection unit 43. The light source unit 41 includes a white LED 44 as a white light source, and a plurality of laser light sources (LD) 45. In this embodiment, as the plurality of laser light sources 45, a red laser light source 45R, a green laser light source 45G, and a blue laser light source 45B are arranged. Note that even if another light source such as a Xenon lamp and a halogen lamp is used as the white light source, the present technology is applicable.

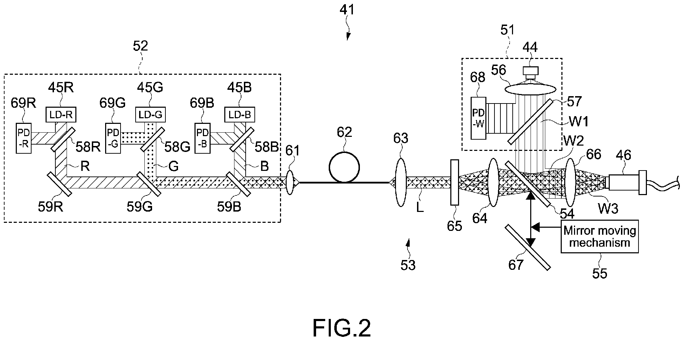

The lights emitted from the white LED 44 and the plurality of laser light sources 45 are multiplexed by a multiplexing optical system 53 (see Fig. 2), and the multiplexed light is emitted to a light guide (optical fiber) 46 connected to the light source unit 41. Then, the light is guided to an illumination port 12 arranged at the front portion 11 of the insert unit 10 through a light guide 46. The light guide 46 and irradiation port 12 function as the irradiation unit in this embodiment. Note that light source unit 41 will be described later referring to Fig. 2 in detail.

The light detection unit 43 can detect intensities of the lights emitted from the white LED 44 and the respective light sources 45. The intensities of the lights are fed back to the control unit 42. Also, the light detection unit 43 will be described later referring to Fig. 2.

The control unit 42 controls start and stop of the operation of the white LED 44 and the respective laser light sources 45, and controls outputs (light outputs) of the white LED 44 and the respective laser light sources 45. Specifically, control unit 42 controls an amount of the current input to each of the white LED 44 and the respective laser light sources 45. The control unit 42 sets a predetermined current value, and a current is applied to each of the white LED 44 and the respective laser light sources 45 by driving circuits (not shown) on the basis of the set value.

In addition, the control unit 42 can execute an APC (Automatic Power Control: light output automatic control) or the like on the white LED 44 and the respective laser light sources 45 on the basis of each intensity of the emitted light fed-back from the light detection unit 43. Also, on the basis of the feedback results, the respective failures of the white LED 44 and the respective laser light sources 45 can be detected.

As the control unit 42, a microprocessor or the like including, for example, a CPU, memories (RAM, ROM), I/O (Input/Output), and the like in one chip. The above-described device such as FPGA and ASIC may be used. The control unit 42 functions as a light source control unit and a detection unit in this embodiment, and the operations thereof will be described later.

Fig. 2 is a schematic diagram showing a specific configuration example of the light source unit 41 shown in Fig. 1. The light source unit 41 includes a first light emitting unit 51, a second light emitting unit 52, a multiplexing optical system 53, a dichroic mirror 54 as the filter unit, and a mirror moving mechanism 55.

At the first light emitting unit 51, the white LED 44 is arranged. In addition, at the first light emitting unit 51, a collimate optical system 56 and a half mirror 57 are arranged. The collimate optical system 56 substantially parallelizes the white light W1 emitted from the white LED 44. The half mirror 57 demultiplexes the substantially parallelized white light W1. In this embodiment, the white light W1 transmitted through the half mirror 57 is emitted toward the dichroic mirror 54 in the downstream.

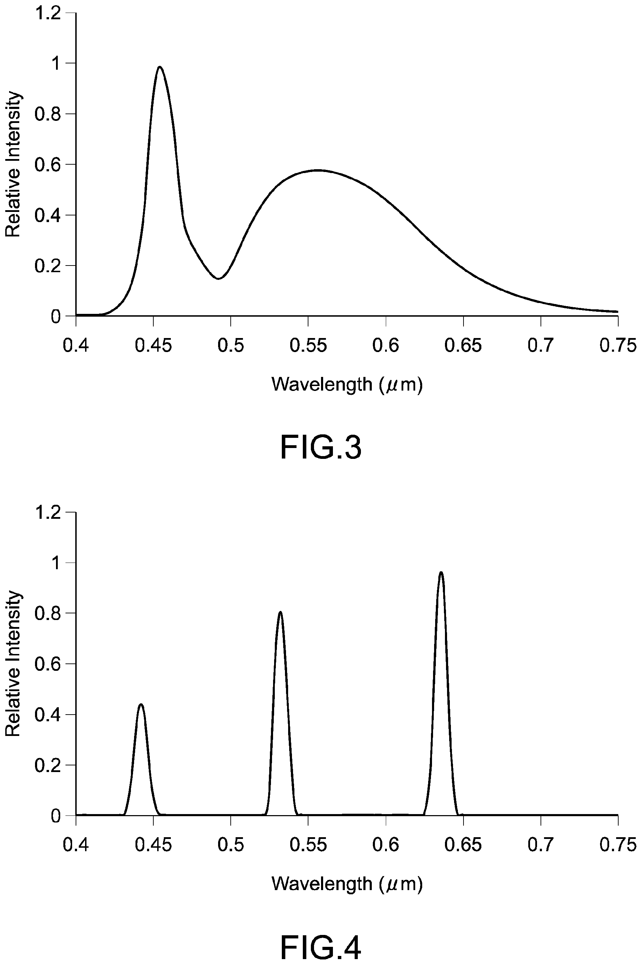

Fig. 3 is a graph showing an example of the spectrum of the white light W1 emitted from the first light emitting unit 51. As shown in the graph, the white light W1 is wide wavelength band light.

At the second light emitting unit 52, the red laser light source 45R, the green laser light source 45G, and the blue laser light source 45B are arranged. Also, at the second light emitting unit 52, three half mirrors 58R, 58G, and 58B and three dichroic mirrors 59R, 59G, and 59B are arranged. The half mirrors 58R, 58G, and 58B demultiplex laser lights R, G, and B emitted from the respective laser light sources 45R, 45G, and 45B.

The dichroic mirror 59R reflects the light having the wavelength band corresponding to the red laser light R emitted from the red laser light source 45R. The dichroic mirror 59G reflects the light having the wavelength band corresponding to the green laser light G emitted from the green laser light source 45G. The dichroic mirror 59B reflects the light having the wavelength band corresponding to the blue laser light B emitted from the blue laser light source 45B.

As shown in Fig. 2, the dichroic mirrors 59R, 59G, and 59B multiplex the laser lights R, G, and B emitted from the respective laser light sources 45R, 45G, and 45B and transmitted through the half mirrors 58R, 58G, and 58B. The multiplexed light (hereinafter described as "laser multiplexed light") L is emitted toward the multiplexing optical system 53 in the downstream.

Fig. 4 is a graph showing an example of the spectrum of the laser multiplexed light L emitted from the second light emitting unit 52. In this embodiment, the red laser light R having a center wavelength of about 638 nm, the green laser light G having a center wavelength of about 532 nm, and the blue laser light B having a center wavelength of about 450 nm are emitted. Note that the center wavelength of each laser light is not limited. The intensities of the respective laser lights (Relative Intensity in the graph) can be independently controlled by the control unit 42, respectively.

The multiplexing optical system 53 includes a coupling optical system 61, an optical fiber 62, first and second collimate optical systems 63 and 64, a diffusion member 65, and a capacitor optical system 66. The coupling optical system 61 optically couples the laser multiplexed light L emitted from the second light emitting unit 52 to an entering end of the optical fiber 62.

The optical fiber 62 leads the entering laser multiplexed light L to the first collimate optical system 63. The emitted light from the optical fiber 62 forms a rotationally symmetric beam. Accordingly, the laser multiplexed light L lead by the optical fiber 62 has a uniform in-plane luminance distribution.

The first collimate optical system 63 substantially parallelizes the laser multiplexed light L emitted from the optical fiber 62. This allows a diffusion status of the laser multiplexed light L to be easily controlled in the diffusion member 65 disposed in the downstream.

The diffusion member 65 diffuses the substantially parallelized laser multiplexed light L, to thereby constitute a secondary light source. Angles of the lights emitted from the optical fiber 62 are generally different depending on the respective laser lights. The divergence angles become constant when the respective laser lights pass through the diffusion member 65. As a result, color unevenness is decreased when the laser multiplexed light L is irradiated.

The second collimate optical system 64 substantially parallelizes the laser multiplexed light L diffused by the diffusion member 65, and emits the laser multiplexed light L toward the dichroic mirror 54 arranged in the downstream. The capacitor optical system 66 forms an image of the light emitted from the dichroic mirror 54 (multiplexed white light W3 obtained by multiplexing attenuated white light W2 and the laser multiplexed light L as described later) at an entering end of the light guide 46.

The specific configurations of the above-described respective optical systems and optical members are not limited. For example, a plurality of optical members and the like may be used as appropriate to realize the above-described optical systems and the like.

The dichroic mirror 54 functioning as the filter unit is arranged on a light path of the white light W1 emitted from the first light emitting unit 51, and is arranged between the second collimate optical system 64 and the capacitor optical system 66 of the multiplexing optical system 53. The dichroic mirror 54 reflects the white light W1 emitted from the first light emitting unit 51 toward the capacitor optical system 66. Also, the dichroic mirror 54 transmits the laser multiplexed light L emitted from the second light emitting unit 52 toward the capacitor optical system 66.

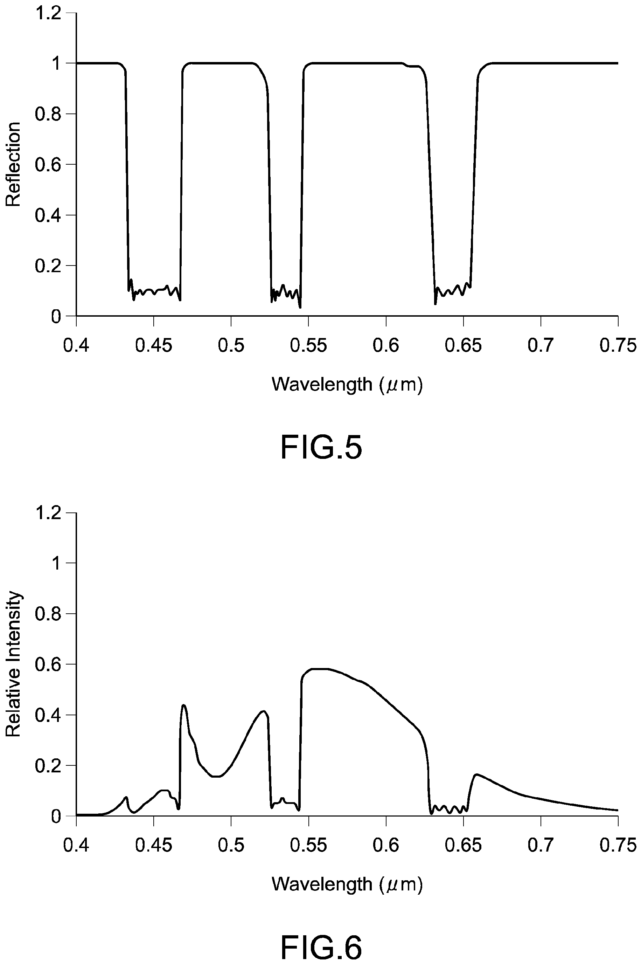

Fig. 5 is a graph showing an example of a reflection property of the dichroic mirror 54. As shown in the graph, the dichroic mirror 54 reflects a major part of the wavelength band of the white light W1, and transmits the lights having the wavelength bands corresponding to the respective laser lights RGB emitted from the second light emitting unit 52. In other words, in this embodiment, the dichroic mirror 54 transmits the light having a predetermined wavelength band including about 638 nm being a center wavelength of the red laser light R, the light having a predetermined wavelength band including about 532 nm being a center wavelength of the green laser light G, and the light having a predetermined wavelength band including about 450 being is a center wavelength of the blue laser light B.

Fig. 6 is a graph showing an example of the spectrum of the white light W1 reflected by the dichroic mirror 54. As shown in the graph, the white light W1 reflected by the dichroic mirror 54 includes attenuated respective wavelength band components of the respective laser lights RGB emitted from the second light emitting unit 52. Hereinafter, this light will be referred to as attenuated white light W2.

Thus, the dichroic mirror 54 has a function to attenuate or remove the light components of the wavelength bands corresponding to the respective laser lights RGB emitted from the second light emitting unit 52 from the white light W1 emitted from the first light emitting unit 51. Also, the dichroic mirror 54 has a function to multiplex the attenuated white light W2 from which the light components of the wavelength bands corresponding to the respective laser lights are attenuated or removed and the respective laser lights RGB (laser multiplexed light L) emitted from the second light emitting unit 52. Hereinafter, the light obtained by multiplexing the attenuated white light W2 and the laser multiplexed light L will be referred to as multiplexed white light W3.

In this embodiment, a multiplex unit is constituted of the multiplex optical system 53 and the dichroic mirror 54, a mutual arrangement relationship being established. Alternatively, the multiplexing optical system 53, which is arranged as appropriate with respect to the dichroic mirror 54, can be considered as the multiplex unit.

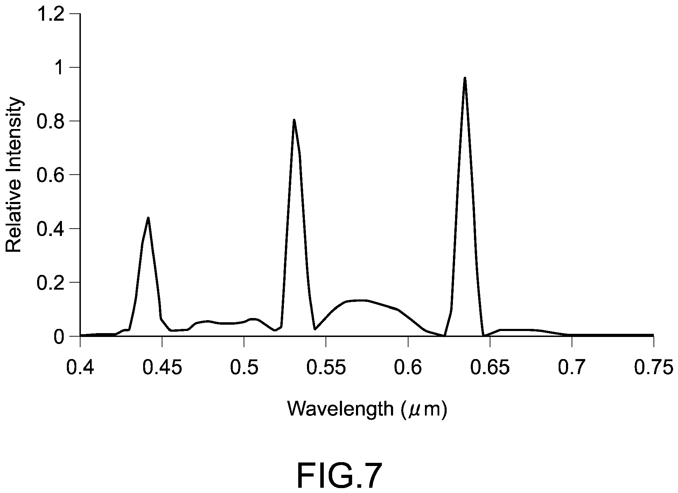

Fig. 7 is a graph showing an example of the spectrum of the multiplexed white light W3. As shown in the graph, the multiplexed white light W3 is a wide wavelength band light including not only the respective laser lights RGB but also wavelength band lights therebetween. As a result, the spectrum of the multiplexed white light W3 can be closer to the spectrum of the white light W1 shown in Fig. 3 or the white light emitted from a halogen lamp, or the like.

In addition, by controlling the outputs of the respective laser lights RGB, it is possible to easily adjust a color temperature of the multiplexed white light W3. For example, even in a case where the respective laser lights R, G, and B are multiplexed with the white light W1, by controlling the output of the respective laser lights, it is possible to adjust the color temperature. On the other hand, as in this embodiment, in a case where the respective laser lights R, G, and B are multiplexed with the attenuated white light W2 reflected by the dichroic mirror 54, the color temperature can be controlled with high accuracy.

Specifically, the respective laser lights RGB according to this embodiment can be used as a plurality of laser lights that can adjust the color temperature of the white light W1 emitted from the white LED 44. It should be appreciated that the respective laser lights R, G, and B may be emitted for the purpose other than adjustment of the color temperature.

In addition, by setting properties, an arrangement position, or the like of each optical member of the multiplexing optical system 53 shown in Fig. 2 as appropriate, the image of the diffusion member 65 constituting the secondary light source and the diameter of the entering end of the light guide 46 can be set to the substantially same. This allows the generation of speckle noises to be sufficiently decreased when the multiplexed white L3 is emitted, for example, to the region to be observed within a body cavity of a patient via the light guide 46.

The mirror moving mechanism 55 removes the dichroic mirror 54 from the light path of the white light W1 and to arrange a total reflection mirror 67 instead. The mirror moving mechanism 55 will be described later in detail.

As shown in Fig. 2, a photodetector 68 is arranged at the first light emitting unit 51. The photodetector 68 receives the white light W1 demultiplexed by the half mirror 57 and detects its intensity.

Photodetectors 69R, 69G, and 69B are arranged corresponding to the respective laser light sources 45R, 45G, and 45B at the second light emitting unit 52. The photodetectors 69R, 69G, and 69B detect each intensity of the laser lights R, G, and B demultiplexed by the half mirrors 58R, 58G, and 58B. As the photodetectors, known devices, e.g., photodiodes or the like, may be used.

The photodetectors 68, 69R, 69G, and 69B constitute the light detection unit 43 shown in Fig. 1. Note that the laser multiplexed light L emitted from the second light emitting unit 52 may be demultiplexed by the half mirror or the like, and the light may be split into three color lights RGB to detect respective intensities. Otherwise, in order to detect the intensities of the white light W1 and the respective laser lights R, G, and B, any configurations and methods may be used.

### [Operations in a case of failure of light sources]

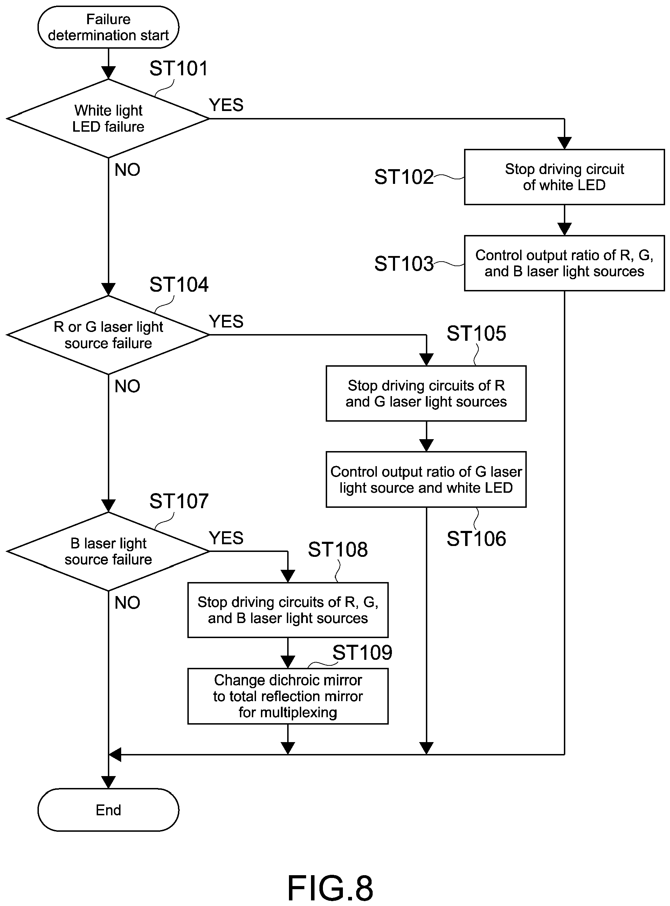

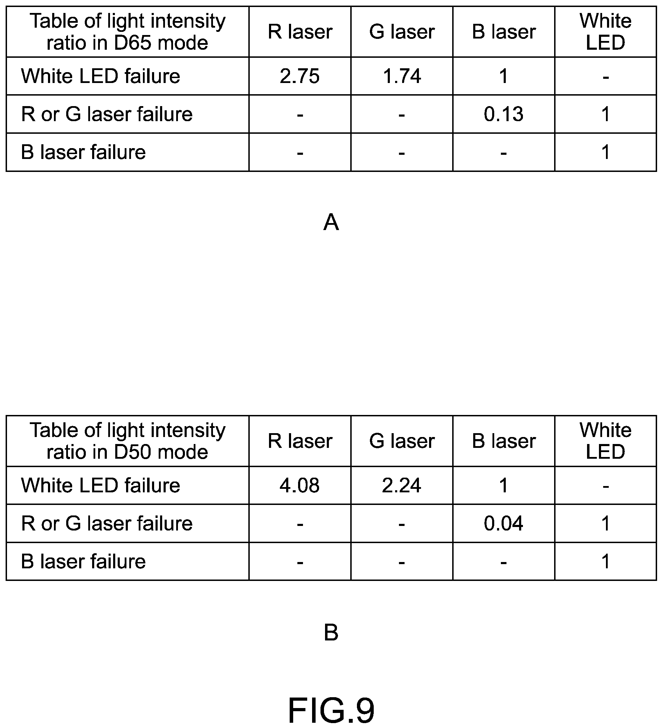

Operations in a case of failure of the white LED 44 and the plurality of laser light sources R, G, and B will be described. Fig. 8 is a flow chart showing an example of the control in a case of failure of the respective light sources. Fig. 9 shows tables each indicating an example of intensity ratios of the lights output from the remaining non-failed light sources.

Presence or absence of the failure of the white LED 44 is determined on the basis of a feedback result from the light detection unit 43 by the control unit 42 functioning as the detection unit (Step 101). For example, the light intensities that are at least expectable are stored in the memory of the control unit 42. The control unit 42 compares the failure determination reference value with the light intensities detected by the light detection unit 43. Then, in a case where the detected value is below the failure determination reference value, the control unit 42 detects the failure of the white LED 44 and determines that the failure occurs.

The method of detecting the failure is not limited. For example, it may be determined that the failure occurs in a case where the current value or the voltage value applied to the white LED 44 is monitored and the monitor result is different from the normal value, or the like. Otherwise, arbitrary technologies may be used.

In a case where it is determined that the white LED 44 has a failure (Yes in Step 101), the control unit 42 that functions as the light source control unit stops the driving circuit of the white LED 44 (Step 102). Also, by controlling the driving current, the output ratio of the respective laser light sources 45R, 45G, and 45B is controlled. Note that the output ratio of the light sources corresponds to the intensity ratio of the lights outputted from light sources.

Fig. 9A is an intensity ratio table showing an example of the intensity ratio of the white light W1 and the respective laser lights R, G, and B in a D65 mode. Fig. 9B is an intensity ratio table showing an example of the intensity ratio of the white light W1 and the respective laser lights R, G, and B in a D50 mode.

The D65 mode is a mode that uses the white light of the D65 standard light source as a standard, and irradiates the region to be observed with white light having a color temperature substantially equal to the color temperature of the standard white light. The D50 mode is a mode that uses the white light of the D50 standard light source as a standard, and irradiates the region to be observed with white light having a color temperature substantially equal to the color temperature of the standard white light. Each mode is selected by an operator's input via the input apparatus 70 shown in Fig. 1, for example.

As shown in Fig. 9A, in a case where the D65 mode is selected, the outputs of the respective laser light sources 45 are controlled such that the following intensity ratio of the respective laser lights RGB is obtained at the time of failure of the white LED 44.
R laser light:G laser light:B laser light = 2.75:1.74:1

As shown in Fig. 9B, in a case where the D50 mode is selected, the outputs of the respective laser light sources 45 are controlled such that the following intensity ratio of the respective laser lights RGB is obtained at the time of failure of the white LED 44.
R laser light:G laser light:B laser light = 4.08:2.24:1

Specifically, in this embodiment, in a case where the failure of the white LED 44 is detected, the intensity ratio of the respective laser lights RGB is controlled. In this regard, instead of the white light W1 from the white LED 44, the white light (laser multiplexed light L) corresponding to each mode is emitted. It should be appreciated that it is not limited to the white light having the color temperature in each of the D65 mode and the D50 mode.

Thus, as different from the control of turning on the special light source in order to draw the scope off as described in the Patent Literature 1, observation, inspection, diagnosis, or the like of the operating field can be continued without interruption. Then, at the time of ending the observation or the like or at an appropriate timing, the insert unit 10 can be safely drawn off from the body cavity. As a result, the effect of the failure of the white LED 44 on the operator's operation can be sufficiently suppressed.

For example, if the insert unit 10 should be drawn off every time the white LED 44 has a failure, the burden on the patient will be considerably great. In this embodiment, as the intensity ratio of the laser lights RGB is adjusted and the observation or the like can be continued, the burden on the patient can be significantly reduced.

Also, as the intensity ratio of the laser lights RGB is adjusted and the white light is irradiated in accordance with the mode, no special image processing on the observation image acquired by the image sensor 14 is necessary. For example, it is assumed that predetermined image processing is executed on the observation image in a case where both of the white LED 44 and the respective laser light sources 45 have no failure, for example. In a case where the white LED 44 has a failure under the state and the special light source as described in Patent Literature 1 is used, it is necessary to change the mode of the image processing on the observation image.

In contrast, in this embodiment, the change of the mode of the image processing is unnecessary, the load to the system controller 31 and a video processor (not shown) can be reduced. Also, in a case where the white LED 44 has a failure, no display delay of the observation image occurs by the change of the image processing mode.

In Step 101, in a case where no failure of the white LED 44 is detected (No), it is determined whether or not the red laser light source 45R or the green laser light source 45G has a failure (Step 104). In other words, it is determined which of the red laser light source 45R and green laser light source 45G has a failure. Note that the detection of the failures of the respective laser light sources 45 RGB is executed similar to the detection of the failure of the white LED 44 as described above.

In a case where it is determined that either the red laser light source 45R or the green laser light source 45G has a failure (Yes in Step 104), the driving circuits of both of the red laser light source 45R and green laser light source 45G are stopped (Step 105). In this regard, the output ratio of the blue laser light source 45B and the white LED 44 is controlled.

As shown in Fig. 9A, in a case where the D65 mode is selected, the outputs of the blue laser light source 45B and the white LED 44 are controlled such that the intensity ratio of the blue laser light B and the white light W1 is as follows:
B laser light:white light W = 0.13:1

As shown in Fig. 9B, in a case where the D50 mode is selected, the outputs of the blue laser light source 45B and the white LED 44 are controlled such that the intensity ratio of the blue laser light B and the white light W1 is as follows:
B laser light:white light W = 0.04:1

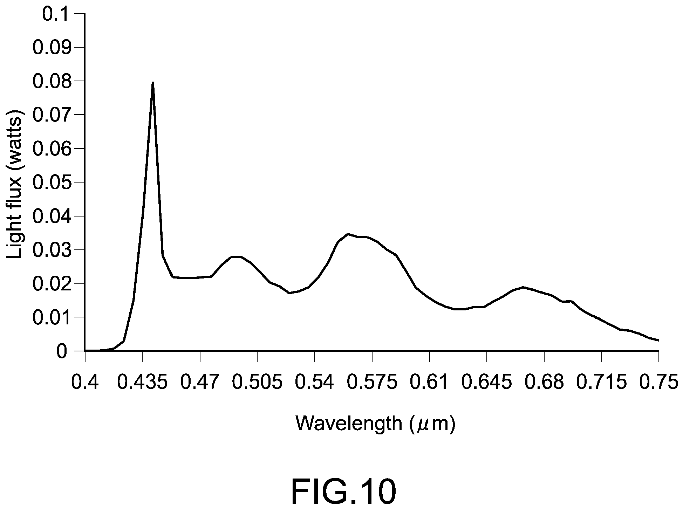

Fig. 10 is a graph showing an example of a spectrum of multiplexed light of the attenuated white light W2 reflected by the dichroic mirror 54 and the blue laser light B (hereinafter described as second multiplexed white light). Note that a radiant flux (watts) is used as a light flux in the vertical axis of the graph.

As shown in Fig. 6, the attenuated white light W2 according to this embodiment is a yellowish colored light. Accordingly, as shown in Fig. 10, by multiplexing the blue laser light B with the attenuated white light W2, the white light (second multiplexed white light) corresponding to each mode can be emitted. As a result, even in a case where either the red laser light source 45R or the green laser light source 45G has a failure, the effect can be sufficiently suppressed. Also, the effect can be exerted similar to the case that the white light is generated from the above-described respective laser lights RGB.

In a case where no failures of the red laser light source 45R and the green laser light source 45G are detected in Step 104 (No), presence or absence of the failure of the blue laser light source 45B is determined (Step 107). In a case where the failure of the blue laser light source 45B is detected (Yes in Step 107), all the driving circuits of the respective laser light sources RGB 45 are stopped (Step 108). Specifically, only the white LED 44 is driven.

In this regard, the mirror moving mechanism 55 shown in Fig. 2 removes the dichroic mirror 54 for multiplexing functioning as the filter unit from the light path of the white light W1, and the total reflection mirror 67 is arranged instead. In other words, the dichroic mirror 54 arranged on the light path of the white light W1 is switched to the total reflection mirror 67 (Step 109).

The total reflection mirror 67 reflects the light having all the wavelength bands included in the white light W1. Accordingly, the white light W1 having the spectrum shown in Fig. 3 is reflected toward the entering end of the light guide 46. Thus, the region to be observed is irradiated with the white light W1 via the light guide 6. As a result, even in a case where the blue laser light source 45B has a failure, the effect can be sufficiently suppressed.

It is thus difficult to emit the white light in the D65 mode, the D50 mode, or the like, or the white light having the adjusted color temperature. Except that point, the above-described effects can be exerted including a continuation of observation or the like, a safe draw-off of the insert unit, a reduction of the burden on the patient, a reduction of the load on the image processing, and the like.

Switching from the dichroic mirror 54 to the total reflection mirror 67 is automatically executed by the control unit 42, for example. In this case, the control unit 42 and the mirror moving mechanism 55 correspond to the filter driving unit according to this embodiment. On the other hand, the mirror moving mechanism 55 may have a lever or the like, and the operator may operate the lever or the like to thereby execute switching of the mirror manually. In this case, the mirror moving mechanism 55 corresponds to the filter driving unit according to this embodiment. In a case where the mirror is switched manually, the GUI that encourages the lever operation or the like may be displayed on the display apparatus 80, an operation panel (not shown), or the like depending on the detection of the failure of the blue laser light source 45B.

Note that removing the dichroic mirror 54 form the light path of the white light W1 is not limited to the case that the dichroic mirror 54 is removed from the light path, and includes the case that the light path of the white light W1 itself is moved while the position of the dichroic mirror 54 is unchanged. For example, the operation of the filter driving unit according to the present technology may include a case where the total reflection mirror 67 is arranged in front of the dichroic mirror 54 or the like.

In Step 107, in a case where no failure of the blue laser light source 45B is detected (No), it is determined that there is no failure and irradiation with the normal white light W3 is executed by the white LED 44 and the respective laser light sources 45.

In this embodiment, the blue laser light source 45B corresponds to the first laser light source. In addition, the red laser light source 45R and green laser light source 45G correspond to the second laser light sources. Specifically, in a case where the first laser light source has a failure, driving of all the plurality of laser light sources is stopped, and the white light source is only driven. Also, in a case where one of the second laser light sources has a failure, driving of this second laser light source and the other second laser light source is stopped, and the first laser light source and the white light source are driven. In this manner, by changing the control upon the failure depending on the types of the laser light sources, it is possible to meticulously deal with the failures of the respective laser light sources and to improve the operability.

Setting the first laser light source and the second laser light sources from the plurality of laser light sources may be made as appropriate. For example, it can be set as appropriate on the basis of the spectrum of the white light W1 emitted from the white light source, attenuated properties of the filter unit (spectrum of the filtered and attenuated white light W2) or the like. Typically, the laser light source emitting laser light that can provide a desirable white light by multiplexing with the attenuated white light W2 is set as the first laser light source. The other laser light sources are set as the second laser light sources. The desirable white light may include light having some tints and being not pure white.

As above, according to this embodiment, even in a case where any light source among the white LED 44 and the respective laser light sources 45 has a failure, it is possible to irradiate the region to be observed with the white light and to sufficiently suppress the effect on the operator's operation.

### <Second embodiment>

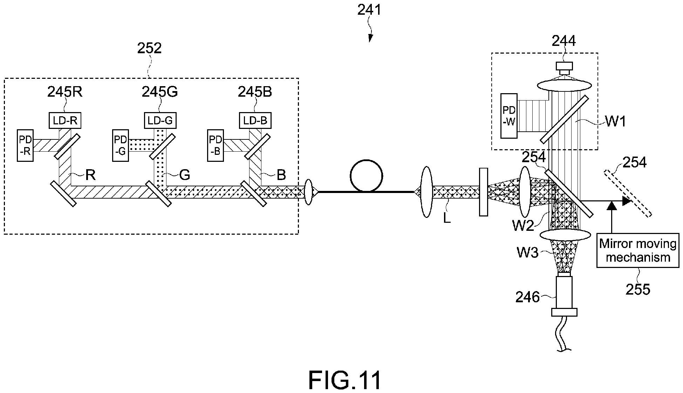

Fig. 11 is a schematic diagram showing a configuration example of a light source apparatus according to a second embodiment. In the description below, in relation to the configuration and the action of the light source apparatus 40 as described in the above embodiment, a detailed description thereof will be hereinafter omitted.

A light source unit 241 included in the light source apparatus according to this embodiment uses a dichroic mirror 254 functioning as the filter unit having properties opposite to the dichroic mirror 54 of the first embodiment. In other words, the dichroic mirror 254 according to this embodiment is designed to have transmission properties shown by the graph of Fig. 5.

Accordingly, the dichroic mirror 254 transmits a major part of the wavelength band of the white light W1, and reflects the wavelength band light corresponding to the respective laser lights RGB emitted from the second light emitting unit 252. As shown in Fig. 11, the attenuated white light W2 transmitting the dichroic mirror 254 and the laser multiplexed light L reflected by the dichroic mirror 254 are multiplexed, and the multiplexed white light W3 is emitted to the entering end of the light guide 246.

In this light source apparatus, in a case where the failure of the blue laser light source 245B is detected (corresponds to Yes in Step 107 of Fig. 8), all the driving circuits of the respective laser light sources RGB 245 are stopped. Then, the dichroic mirror 254 is removed from the light path of the white light W1 by the mirror moving mechanism 255.

In other words, as different from the first embodiment in which the mirror is switched, by only removing the dichroic mirror 254, the white light W1 from the white LED 244 can enter the light guide 246. As a result, the configuration of the mirror moving mechanism 255 can be simplified, and the size of the apparatus can be reduced. Note that the dichroic mirror 254 can be removed automatically or manually.

### <Other embodiments>

The present technology is not limited to the above-described embodiments, and other various embodiments may be implemented.

The mode of irradiating with the multiplexed white light as described above is taken as a normal observation mode. A special observation mode different from the normal observation mode may be executable. Examples of the special observation mode include a mode of executing Photodynamic Diagnosis (PDD) and Photodynamic Therapy (PDT), for example.

The PDD and PDT are less invasive tumor diagnosis and treatment methods using a light-sensitive drug, and are applied to diagnosis and treatment of early stage lung cancer, early stage esophagus cancer, stomach cancer, early stage cervical cancer, brain malignancy, or the like. The PDD diagnoses a tumor site or the like by utilizing the properties of the light-sensitive drug, which is excited by excitation light having a predetermined wavelength band and emits fluorescence. The PDT treats the tumor site or the like by utilizing the properties of the light-sensitive drug that generates active oxygen by irradiation with the excitation light having the predetermined wavelength band.

In the special observation mode of the PDD, the PDT, or the like, the respective laser light sources RGB shown in Fig. 1 or the like may be used as the excitation light sources. In addition to the respective laser light sources RGB, the excitation light source such as an IR light source for Indocyanine Green (ICG) fluorescence observation and a 405 nm light source for 5-aminolevulinic acid (5-ALA) fluorescence observation may be additionally arranged. Also, the excitation light source arranged as the light source for the excitation light may be used to adjust the color temperature of the white light from the white light source. In this case, the plurality of laser light sources according to the present technology includes the excitation light source as one laser light source. Also, in this case, the filter unit is configured such that the light having the wavelength band corresponding to the excitation light emitted from the excitation light source can be attenuated.

As the configuration that the special observation mode is executable, the excitation light source may be arranged at the first light emitting unit. In this case, in the special observation mode, for example, only the excitation light source is driven, and the filter unit is removed from the light path. Alternatively, the color temperature of the excitation light may be adjusted by the plurality of laser light sources arranged at the second light emitting unit. In this case, the filter unit may be arranged as it is.

In the above, the respective laser light sources RGB corresponding to three-primary-color lights are arranged at the second light emitting unit. However, it is not always limited to this configuration. In a case where the white light source has a failure, the number or the types (wavelength band of emitted light) of the laser light sources may be set as appropriate within the range that the desirable white light is provided in the remaining plurality of laser light sources.

In the above, the dichroic mirror having a wavelength filter function is used as the filter unit. But it is not limited thereto, and another optical member such as a dichroic prism may be used. Also, the method of stopping an attenuating function of the filter unit is not limited to the removal of the filter unit from the light path of the white light. Any other methods may be used.

The present technology is applicable not only to the endoscope apparatus but also to other apparatuses and other systems in the medical and biological fields including optical microscopes. Otherwise, the light source apparatus and the light source driving method according to the present technology may be applied to an apparatus and a system in a variety of fields including an optical recording system, a semiconductor exposure apparatus, and the like.

Among the features according to the present technology as described above, at least two features may be combined. In other words, a variety of the features described in the respective embodiments may be arbitrarily combined interchangeably. Also, a variety of the effects described in the above are merely illustrative and are not limitative, and other effects may be exerted.

The present technology may also have the following configurations.
(1) A light source apparatus, including:
   a white light source that emits white light;
   a plurality of laser light sources that emit a plurality of laser lights capable of adjusting a color temperature of the white light;
   a detection unit capable of detecting a failure of each of the white light source and the plurality of laser light sources; and
   a light source control unit that controls an intensity ratio of the plurality of laser lights that are emitted from the plurality of laser light sources in a case where the failure of the white light source is detected.
(2) The light source apparatus according to (1), in which
   the light source control unit that emits the white light from the white light source in a case where the failure of any of the plurality of laser light sources is detected.
(3) The light source apparatus according to (1) or (2), further including:
   a filter unit that attenuates each of wavelength band components of the plurality of laser lights; and
   a multiplex unit that multiplexes the white light and the plurality of laser lights, the white light being the white light where each of wavelength band components of the plurality of laser lights is attenuated by the filter unit.
(4) The light source apparatus according to (3), in which
   the plurality of laser light sources include a first laser light source and a second laser light source different from the first laser light source, and
   the light source control unit drives only the white light source in a case where the failure of the first laser light source is detected, and drives the first laser light source and the white light source in a case where the failure of the second laser light source is detected.
(5) The light source apparatus according to (4), further including:
   a filter driving unit capable of removing the filter unit from a light path of the white light emitted from the white light source in a case where the failure of the first laser light source is detected.
(6) The light source apparatus according to any one of (1) to (5), in which
   the white light source is a white LED (Light Emitting Diode), and
   the first laser light source of the plurality of laser light sources includes a blue laser light source, and the second laser light source of the plurality of laser light sources includes a red laser light source and a green laser light source.

### Reference Signs List

R red laser light
G green laser light
B blue laser light
L laser multiplexed light
W1 white light
W2 attenuated white light
W3 multiplexed white light
40 light source apparatus
41, 241 light source unit
42 control unit
43 light detection unit
44 white LED
45 plurality of laser light sources (LD)
53 multiplexing optical system
54, 254 dichroic mirror
55, 255 mirror moving mechanism
67 total reflection mirror
100 endoscope apparatus

## Claims

1. A light source apparatus, comprising:
a white light source that emits white light;
a plurality of laser light sources that emit a plurality of laser lights capable of adjusting a color temperature of the white light;
a detection unit capable of detecting a failure of each of the white light source and the plurality of laser light sources; and
a light source control unit that controls an intensity ratio of the plurality of laser lights that are emitted from the plurality of laser light sources in a case where the failure of the white light source is detected.

2. The light source apparatus according to claim 1, wherein
the light source control unit that emits the white light from the white light source in a case where the failure of any of the plurality of laser light sources is detected.

3. The light source apparatus according to claim 1, further comprising:
a filter unit that attenuates each of wavelength band components of the plurality of laser lights; and
a multiplex unit that multiplexes the white light and the plurality of laser lights, the white light being the white light where each of wavelength band components of the plurality of laser lights is attenuated by the filter unit.

4. The light source apparatus according to claim 3, wherein
the plurality of laser light sources include a first laser light source and a second laser light source different from the first laser light source, and
the light source control unit drives only the white light source in a case where the failure of the first laser light source is detected, and drives the first laser light source and the white light source in a case where the failure of the second laser light source is detected.

5. The light source apparatus according to claim 4, further comprising:
a filter driving unit capable of removing the filter unit from a light path of the white light emitted from the white light source in a case where the failure of the first laser light source is detected.

6. The light source apparatus according to claim 1, wherein
the white light source is a white LED (Light Emitting Diode), and
the first laser light source of the plurality of laser light sources includes a blue laser light source, and the second laser light source of the plurality of laser light sources includes a red laser light source and a green laser light source.

7. A light source driving method, comprising:
detecting a failure of each of a white light source that emits white light and a plurality of laser light sources that emit a plurality of laser lights capable of adjusting a color temperature of the white light; and
controlling an intensity ratio of the plurality of laser lights that are emitted from the plurality of laser lights in a case where the failure of the white light source is detected.

8. The light source driving method according to claim 7, further comprising:
multiplexing attenuated white light and the plurality of laser lights, the attenuated white light being the white light where each of wavelength band components of the plurality of laser lights is attenuated, in a case where no failure of the white light source and the plurality of laser light sources is detected.

9. The light source driving method according to claim 8, further comprising:
driving only the white light source in a case where the failure of a first laser light source among the plurality of laser light sources is detected; and
multiplexing and emitting the attenuated white light and the laser light emitted from the first laser light source in a case where the failure of second laser light source is detected, the second laser light source being different from the first laser light source among the plurality of laser light sources.

10. The light source driving method according to claim 9, further comprising:
removing a filter unit that attenuates each of wavelength band components of the plurality of laser lights from a light path of the white light emitted from the white light source in a case where the failure of the first laser light source is detected.

11. An observation apparatus, comprising:
a light source apparatus, including
a white light source that emits white light,
a plurality of laser light sources that emit a plurality of laser lights capable of adjusting a color temperature of the white light,
a detection unit capable of detecting a failure of each of the white light source and the plurality of laser light sources, and
a light source control unit that controls an intensity ratio of the plurality of laser lights that are emitted from the plurality of laser light sources in a case where the failure of the white light source is detected; and
an irradiation unit that irradiates a region that is a target to be observed with light emitted from the light source apparatus.
